(19) Europäisches Patentamt
European Patent Office
Office européen des brevets

(11) Publication number: 0 365 791 A2

(12) **EUROPEAN PATENT APPLICATION**

(21) Application number: 89116088.9

(51) Int. Cl.5: C07C 37/14

(22) Date of filing: 31.08.89

(30) Priority: 27.10.88 IT 2244488

(43) Date of publication of application:
02.05.90 Bulletin 90/18

(84) Designated Contracting States:
AT BE CH DE ES FR GB GR IT LI LU NL SE

(71) Applicant: SOCIETA' ITALIANA RESINE S.I.R. S.p.A.
Via Grazioli 33
I-20161 Milano(IT)

(72) Inventor: Balducci, Sandro
Via Bellini, 35
I-20050 Macherio Milano(IT)
Inventor: Attanasi, Orazio
Facoltà di Scienze Matematiche-Fisiche e Naturali
I-61029 Urbino(IT)
Inventor: Filippone, Paolino
Facoltà di Scienze Matematiche-Fisiche e Naturali
I-61029 Urbino(IT)

(74) Representative: Perani, Aurelio et al
c/o JACOBACCI-CASETTA & PERANI 7, Via Visconti di Modrone
I-20122 Milano(IT)

(54) **Process for producing an antioxidant from phenolic pitch.**

(57) A method is described for the production of an antioxidant from a phenolic pitch residue remaining after the production of phenol from cumene and including: phenol; 2- and 4-cumyl phenol, acetophenone, dimethylphenylcarbinol; 2,4-diphenyl-4-methyl-1-pentene; 2,4-diphenyl-4-methyl-2-pentene; α-methylstyrene and phenolic derivatives with high molecular weights. The method comprises the alkylation of the phenol, the cumylphenols and the high-molecular-weight phenolic derivatives in the phenolic pitch, with the consequent formation of phenolic antioxidants therein. The antioxidants produced are preferably, but not exclusively, used in the rubber industry.

EP 0 365 791 A2

The present invention relates to a method of producing an antioxidant from a phenolic pitch including phenol, 2- and 4-cumylphenol and phenolic derivatives with high molecular weights.

More particularly this invention concerns a method of producing a substance with antioxidant properties, usable preferably, but not exclusively, in the rubber industry, from phenolic pitch residues resulting from the production of phenol from cumene.

In the description below and in the following claims, the term: phenolic pitch is intended to identify a residual product resulting from the production of phenol from cumene.

In general, the production of phenol from cumene is based on the oxidation of the cumene (I) to the hydroperoxide (II) and the break-down of the latter in an acidic medium to produce phenol (III) and acetone (IV) according to the following reaction scheme:

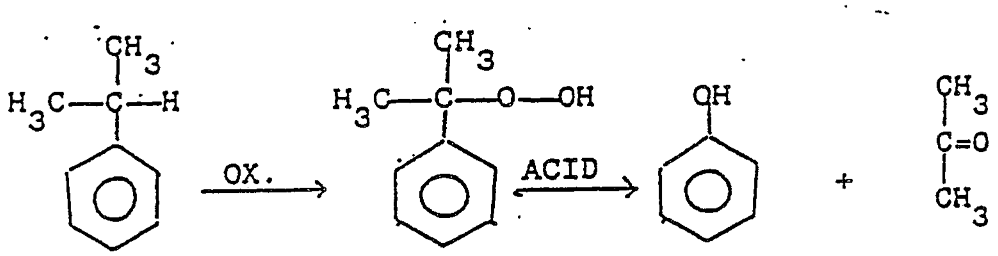


(A)

(I) (II) (III) (IV)

The expected yield of this reaction (A) is generally 93-95%. The phenol and the acetone produced are then separated by fractional distillation giving a residual product of a pitchy nature, the phenolic pitch itself which includes a certain amount of unseparated phenol (III), phenolic aromatic compounds such as 2- and 4-cumylphenol (V), (VI):

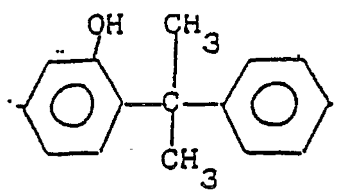

(V)

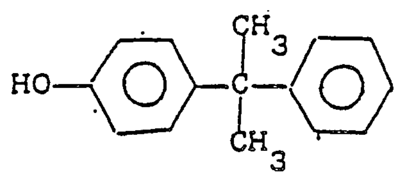

(VI)

and phenolic derivatives with high molecular weights (XIII) of the type including one or more akyl and/or aryl substituents in the aromatic phenol ring, represented schematically by the following structural formula:

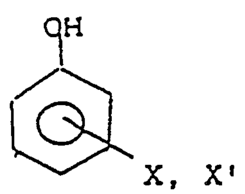

(XIII)

where X, X′ comprise, independently, hydrogen and at least one substituent selected from the group comprising: saturated or unsaturated, linear or branched alkyl groups with from 2 to 6 alkyl carbon atoms, aryl groups with from 6 to 18 aromatic carbon atoms, possibly substituted with hydroxyl groups and/or linear or branched alkyl groups with from 2 to 6 carbon atoms.

The phenolic pitch also includes aromatic compounds of a non-phenolic nature including acetophenone

(VII), dimethylphenylcarbinol (VIII), α-methyl-styrene (XI) and its unsaturated dimers such as 2,4-diphenyl-4-methyl-1-pentene (IX) and 2,4-diphenyl-4-methyl-2-pentene (X):

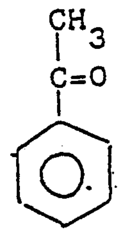

(VII)

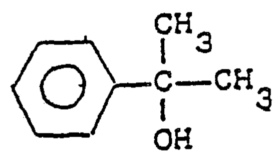

(VIII)

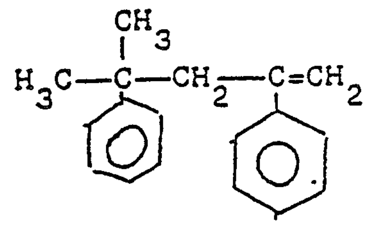

(IX)

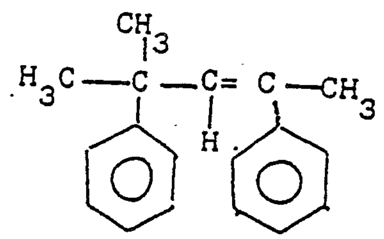

(X)

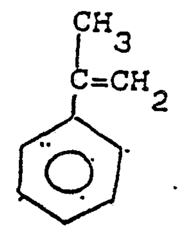

(XI)

The chemical composition of phenolic pitches may vary in dependence on the type of process and the conditions used in the production of the phenol but at any rate, a typical range of values is summarised in table 1 below.

TABLE 1

| Average composition of phenolic pitch resides resulting from the production of phenol from cumene | |
|---|---|
| Compound | % by weight in the pitch |
| Phenol (III) | 3-10 |
| Dimethylphenylcarbinol (VIII) | 4-8 |
| Unsaturated α-methylstyrene dimers (IX-X) | 12-25 |
| Cumylphenols (V, VI) | 15-25 |
| Acetophenone (VII) | 10-25 |
| Phenolic derivatives with high molecular weights (XIII) | 25-45 |

As is known, phenolic pitches have such a degree of toxicity and related pollutant capacity that, in the light of the current legal standards on environmental protection, their disposal constitutes a considerable problem which is difficult to solve.

The seriousness of this problem is further and continually increased by the progressive increase in the world's production of phenol from cumene.

The search for possible treatments for the pitches to lessen their pollutant content in order to allow their disposal as conventional waste has given essentially negative results to the point that it has, for the most part, been abandoned.

The incomplete combustion of the pitch in suitable pyrolisis ovens to produce lampblack has been attempted. This type of solution, carried out by prior art techniques, has, however, found limited application

up to now because of recognised management difficulties and the fact that this treatment is essentially uneconomic.

One prior art method which has, however, had partial success is that based on the concept of finding uses for the phenolic pitch, as such, by making use of some of its chemical and physical characteristics.

According to this method, the phenolic pitch may be used as such (as a plasticizer or as a polymerisation coadjuvant) in bituminous and/or polymeric compositions for coatings for protection against acids or atmospheric agents.

However, this application uses very small quantities of the phenolic pitch so that the seriousness of the problem of its disposal remains substantially unchanged.

Within the scope of the above concept, the present invention provides a method of the type in question, which is characterised in that the phenol (III), the 2-and 4- cumylphenols (V), (VI) and the phenolic derivatives with high molecular weights (XIII) are alkylated in the pitch so as to produce phenolic antioxidants in the pitch itself.

According to one aspect of this invention, the alkylation of the phenol (III), the cumylphenols (V), (VI) and the high-molecular-weight phenolic derivatives (XIII) is, to advantage, carried out with those aromatic compounds, dimethylphenylcarbinol (VIII), 2,4-diphenyl-4-methyl-1-pentene (XI), 2,4-diphenyl-4-methyl-2-pentene (X), α-methylstyrene (XI), which are present in the phenolic pitch and have the structural formulae given above.

According to a variant of the method of the present invention, the akylation of the phenol (III), the cumylphenols (V, VI) and the high-molecular-weight phenolic derivatives (XIII) may be carried out with the addition, to the aromatic alkylating compounds (VIII, IX, X, XI) already present in the phenolic pitch, of any compound or mixture of compounds used industrially as alkylating agents such as for example: olefins, chloroparaffins, alcohols, ethers, possibly substituted with one or more aromatic groups and preferably having from 4 to 18 carbon atoms.

Of these additional alkyating compounds, the preferred ones are those which, in the reaction conditions envisaged, can give rise to carbonium ions or secondary or tertiary radicals, such as for example: isobutene, isopentene, tert-butyl alcohol, tert-butyl chloride, methyl tert-butyl ether, styrene, methylphenylcarbinol.

The alkylation may be carried out by currently-known methods used industrially, which operate according to a Friedel-Crafts or similar reaction mechanism: for this purpose, a quantity of from approximately 2 to 10% by weight, preferably from 4 to 6% by weight, of a Friedel-Crafts catalyst, preferably aluminium chloride, possibly with an acid co-catalyst such as anhydrous hydrogen chloride, is added to the phenolic pitch. The alkylation of the phenol (III), the cumylphenols (V) and (VI) and the high-molecular-weight phenolic derivatives (XIII) is preferably carried out at a temperature within the range 25 to 100 °C. Under these conditions, the akylation reaction is completed in a period of from approximately 24 hours at 25 °C to approximately 3 hours at 100 °C.

For a clearer understanding of the characteristics of the method of the present invention, the antioxidant substances produced thereby comprise a series of compounds generally indicated by the term: phenolic antioxidants.

These latter include substituted phenolic compounds and mixtures thereof, having the following structural formulae:

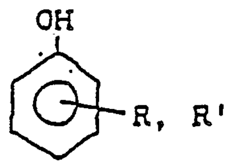

(XIV)

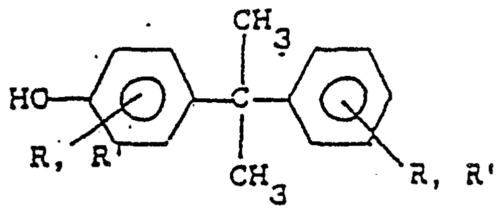

(XV)

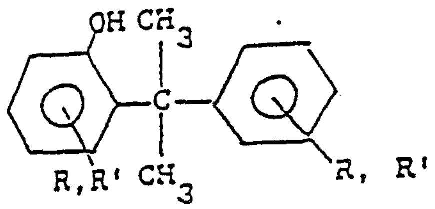

(XVI)

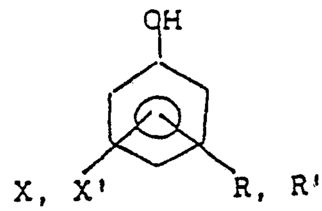

(XVII)

where X, X′ have the meanings given above and R, R′ comprise, independently, hydrogen and at least one substituent selected from the group comprising: isopropyl, sec-butyl, tert-butyl, tert-amyl, sec-ethylbenzyl as well as groups having the following structural formulae:

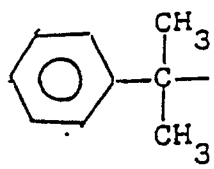

(XVIII)

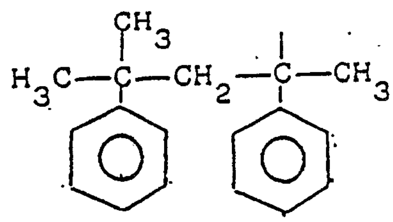

(XIX)

Upon analysis, the phenolic antioxidants obtained by the method of the present invention had the following physical characteristics (average values measured at 25° C):

| | |
|---|---|
| colour | black |
| density | 1.1 - 1.3 kg/$m^3$ |
| Brookfield viscosity | 2,500 - 50,000 centipoise |
| refractive index | 1.5 - 1.7 |

By virtue of the presence of a sterically-hindered hydroxylic functional group, the substituted phenolic compounds (XIV, XV, XVI, XVII) show characteristics, as will become clearer from the description below, which are wholly comparable with those possessed by the phenolic antioxidants commonly used as inhibitors of the radical oxidation of elastomers.

It is, in fact, recognised that the antioxidant activity possessed by phenolic antioxidants is definitely related to the degree of steric hindrance of the hydroxylic functional group.

The method of the present invention thus enables not only the disposal of the phenolic pitch, which is a highly toxic residue, but also its complete and advantageous re-use after conversion into an antioxidant. The latter may be used preferably, but not exclusively, as an antioxidant additive to rubber and elastomeric products.

For this purpose, the phenolic antioxidant obtained by the method of this invention may, to advantage,

be used as it is, or in combination with one or more other antioxidants selected from those currently available on the market.

In a preferred embodiment, the method of the present invention provides a stage in which the acetophenone (VII) is removed from the phenolic pitch before the alkylation stage or from the antioxidant substance obtained therefrom.

In fact, acetophenone (VII), as well as not having any appreciable antioxidant activity and thus reducing the effectiveness of the antioxidant substance in which it is included, has a characteristic penetrating smell which can adversely affect the commercial value of the antioxidant.

The acetophenone can be removed from the phenolic pitch or from the phenolic antioxidant obtained therefrom by fractional distillation, or by stripping, by wholly conventional techniques. The removal of the acetophenone (VII) to advantage enables:

- the viscosity of the pitch or of the phenolic antioxidant to be adjusted to an optimal value,
- the characteristic penetrating smell of the aforesaid ketone to be eliminated, or at least reduced,
- the recovery of the intermediate product, that is the acetophenone which, after purification, can be used as an intermediate in other processes.

In to a further embodiment, however, the method of the invention provides an earlier stage of selective reduction of the acetophenone to monomethylphenylcarbinol (XX), which has the following structural formula:

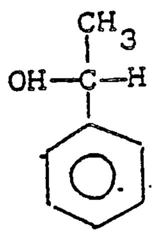

(XX)

The monomethylphenylcarbinol (XX) obtained by the reduction of the acetophenone (VIII) is a further alkylating compound which can be used in the alkylation of the phenol (III), the cumylphenols (V, VI) and the high-molecular-weight phenolic derivatives (XIII). The acetophenone (VII) reduction stage has the advantage not only of eliminating an undesired compound but also of producing a further reagent in the phenolic pitch itself.

This reduction can be carried out, in wholly conventional manner, with the use of sodium borohydride $NaBH_4$ added to the pitch in a proportion of 5-7% by weight, or by means of hydrogenating catalysts of known type such as Raney nickel, in its various modifications, platinum black, etc. As regards the manner in which the acetophenone is reduced, the following literature is given below by way of reference: Italian patent No. 1191868, Kirk Othmer, Encyclopedia of Chemical Technology, vol. 1, page 168.

During the subsequent alkylation of the phenol (III), the cumylphenol (V, VI) and the high-molecular-weight phenolic derivatives (XIII), the methylphenylcarbinol (II) produced enables an aromatic hydrogen atom to be replaced by the following sterically hindered aryl group:

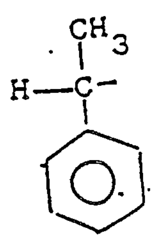

(XXI)

The phenolic antioxidants of the present invention, as will become clearer from the description below, have antioxidant capabilities substantially comparable to those of the antioxidants currently on the market.

Finally, it should be noted that the phenolic antioxidants obtained by the method of the present invention have the further advantage of not altering the colour of the elastomeric product to which they are added, which is particularly important when they are used as antioxidant additives for rubbers and elastomers with low degrees of colouring.

Further characteristics and advantages of the invention will become clearer from the following detailed description of some embodiments of the method according to the invention for the production of an antioxidant from phenolic pitch, given purely by way of non-limiting example

EXAMPLE 1

1056 g of phenolic pitch having the following composition (percentages by weight):

| | |
|---|---|
| Acetophenone | 21 |
| Phenol | 4 |
| Dimethylphenylcarbinol | 5 |
| α-methylstyrene dimers | 21 |
| Cumylphenols | 9 |
| Phenolic derivatives with high molecular weights | 40 |

were loaded into a 3 l distillation flask provided with a stirrer and a reflux condenser, and 44 g of anhydrous aluminium chloride were added.

3 g of anhydrous hydrogen chloride were then bubbled into the reaction mixture over a period of approximately 15 minutes.

The reaction mixture was then brought to a temperature of approximately 50° C and stirred constantly for approximately 6 hours.

After cooling to ambient temperature and neutralisation to pH 6 with 50% NaOH, the reaction product was filtered through a very fine mesh filter, producing a solid residue of approximately 30 g and 1050 g of a phenolic antioxidant whose antioxidant properties are indicated in table 3 below (sample 1).

The physical characteristics of the phenolic antioxidant thus obtained were as follows:

| | |
|---|---|
| colour | black |
| density at 25° C | 1.1128 kg/m$^3$ |
| Brookfield viscosity at 25° C | 2,800 centipoise |
| refractive index at 25° C | 1.5885 |

EXAMPLE 2

1000 g of phenolic antioxidant were prepared in the manner described in Example 1 above.

After filtering through a very fine mesh, the filtrate was distilled under vacuum at approximately 40 Torr to remove the acetophenone present in the pitch and 901 g of phenolic antioxidant (sample 2) and 74 g of acetophenone were produced.

EXAMPLE 3

1000 g of phenolic pitch having the composition given in Example 1 were dissolved in a litre of methanol in a distillation flask having a capacity of approximately 3 l. 60 g of sodium borohydride $NaBH_4$ were added to the resulting solution in small doses at ambient temperature. The additions of sodium

borohydride were made with continuous stirring over approximately two hours in order to enable the acetophenone in the phenolic pitch to be reduced selectively to methylphenylcarbinol (XX). This reduction reaction took place with a yield of approximately 75%.

The resulting solution was then distilled under vacuum at approximately 40 Torr to remove the methanol; the end product of the distillation, containing the pitch, was then dissolved in one litre of methylene chloride and washed successively with washing solutions containing 50 g of 10% sulphuric acid, each washing being followed by a stage in which the aqueous washing phase was separated from the organic phase containing the phenolic pitch.

The latter organic phase was then distilled at 40 Torr to remove the methylene chloride and any residual traces of water and transferred to a 3 l-capacity distillation flask provided with a stirrer and a reflux condenser.

After the addition of 100 g of aluminium chloride the alkylation reaction took place in the same manner as in Example 1.

The reaction product was then cooled to ambient temperature, neutralised with 50% NaOH and filtered, giving 1007 g of a phenolic antioxidant (sample 3) and 72 g of a solid residue.

## EXAMPLE 4

100 g of α-methylstyrene and 44 g of anhydrous aluminium chloride were added to 1056 g of phenolic pitch having the composition given in Example 1. The resulting mixture was then heated to 100° C and kept at that temperature for 3 hours with continuous stirring.

The reaction product was then cooled to ambient temperature, neutralised to pH 5 with 50% NaOH and filtered through a very fine mesh to give 34 g of solid residue and 1162 g of phenolic antioxidant (sample 4).

## EXAMPLE 5

1000 g of phenolic antioxidant were obtained by the method of Example 4 and distilled under vacuum at 40 Torr to remove the approximately 10% of acetophenone which remained substantially unchanged after the alkylation reaction (sample 5).

## EXAMPLE 6

80 g of tert-butyl alcohol and 50 g of aluminium chloride were added to 1000 g of phenolic pitch having the composition indicated in Example 1 and then loaded into a 3 l-capacity distillation flask provided with a stirrer and a reflux condenser. 5 g of anhydrous gaseous hydrogen chloride were bubbled into the reaction mixture as a co-catalyst over a period of approximately 20 minutes. The reaction mixture was then brought to a temperature of approximately 30° C and stirred constantly for approximately 24 hours.

After cooling to ambient temperature and neutralising to pH 6 with 50% NaOH, the reaction product was filtered through a very fine mesh and produced 45 g of solid residue and 1090 g of phenolic antioxidant (sample 6).

## EXAMPLE 7

### Determination of the antioxidant properties

The phenolic antioxidants obtained by means of the preparation examples given above were subjected to several tests to evaluate their antioxidant properties, the properties being compared with those of a

commercial antioxidant widely used in the rubber industry, such as WINGSTAY T (Registered Trade Mark traded by GOODYEAR). The evaluation was carried out by the addition, in a proportion of 2% by weight, of each antioxidant under test, to a natural-rubber-based mixture (NR WHITE) having the composition given in Table 2 below.

TABLE 2

| Composition of the reference mixture | |
|---|---|
| Constituent | (% by weight) |
| NR WHITE natural rubber | 53.5 |
| ZnO | 5.0 |
| Stearic acid | 1.5 |
| Glicogum 4000 | 1.0 |
| Ultrasil UN3 | 35.0 |
| Mercaptobenzothiazole | 1.0 |
| Tetramethylthiuram disulphide | 2 |
| Sulphur | 1 |

The characteristics of resistance to oxidation were evaluated for the various mixtures obtained with the use of the standard qualitative and quantitative methods adopted by the Centro de Ricerca e di Sviluppo dell'Impiego degli Elastomeri C.E.R.I.S.I.E. (Centre for Research and Development into the Use of Elastomers).

A series of mixture samples were prepared with the use of the aforesaid NR WHITE rubber-based mixture and incorporating WINGSTAY T and the phenolic antioxidants prepared according to Examples 1-6 above, in a proportion of 2% by weight, as antioxidising agents.

Each mixture was then vulcanized at 150°C for 5 minutes, the progress of the vulvanization being checked by means of a vulcanograph with a rotor oscillating with an amplitude of + 1°, as prescribed in the table UNI 8342. Plates with dimensions of 200x200x2 mm were formed from the vulcanized rubber samples produced.

During each vulcanization test, the characteristic parameters of the vulcanization, such as the time at which cross-linking started, the time taken to achieve optimum vulcanization, the rate of vulcanization, followed the same type of behaviour both with WINGSTAY T and with the phenolic antioxidants of the present invention.

In order to evaluate the antioxidant properties of the phenolic antioxidants of the present invention and to compare them with those possessed by WINGSTAY T, the changes in their main mechanical characteristics (breaking load, extensibility) were observed after the samples had been subjected to an accelerated ageing treatment.

For this purpose, test pieces were formed from each plate and were subjected to accelerated ageing in a thermostatically-controlled chamber with a natural air circulation for a period of approximately 72 hours at 100°C in accordance with UNI 5408.

Each test piece was then subjected to a conventional tensile test to determine its breaking load and extensibility in accordance with UNI 6065.

The results of the tensile tests are given in Table 3 below, which shows the percentage decreases in the breaking loads and in the extensibilities with respect to reference values, taken as 100, possessed by the reference mixture without any antioxidant agent and without being subjected to any ageing.

TABLE 3

| Antioxidant added | Decrease (%) in breaking load | Decrease (%) in extensibility |
|---|---|---|
| none | 71-75 | 45-48 |
| WINGSTAY T | 36-38 | 30-32 |
| sample 1 | 43-45 | 35-37 |
| sample 2 | 40-42 | 32-35 |
| sample 3 | 36-40 | 30-32 |
| sample 4 | 39-43 | 32-35 |
| sample 5 | 37-41 | 30-33 |
| sample 6 | 40-44 | 33-36 |

As can be seen from the above table, the resistance to oxidation of the mixtures prepared with the phenolic antioxidants of the present invention is substantially comparable to that provided by WINGSTAY T.

Test pieces were then prepared by cutting and punching from the vulcanized rubber plates for use in determination of the characteristics of SHORE hardness, stainability and colour variation.

More particularly, the SHORE hardness was determined in accordance with UNI 4916 on test pieces with a thickness of 4 mm whilst the colour-variation tests were carried out by the exposure of the test pieces to the light of a 500W OSRAM HWL sun lamp at a temperature of 65 ± 5 °C, in accordance with UNI 7097.

All the vulcanized mixtures incorporating the phenolic antioxidants of the present invention gave SHORE hardness values substantially comparable with that of the mixture including WINGSTAY T and showed no colour variation.

It is clear from what has been described and illustrated that the method of the present invention enables, not only the disposal of a toxic residue which is difficult to eliminate, but also the simultaneous, advantageous production of an antioxidant whose characteristics are substantially comparable with those possessed by the antioxidants currently on the market.

In use as antioxidant additives in rubber and elastomers in general, the phenolic antioxidants of the present invention have the further advantage that they do not alter the colour of, nor stain the product to which they are added.

Not the least advantage of the method of the present invention is that it enables an antioxidant with good characteristics to be produced on a commercial scale at low cost.

## Claims

1. A method of producing an antioxidant from a phenolic pitch including phenol (III), 2- and 4-cumylphenol (V), (VI) and phenolic derivatives with high molecular weights (XIII), characterised in that the phenol (III), the 2-cumylphenol (V), the 4-cumylphenol (VI) and the high-molecular-weight phenolic derivatives (XIII) are alkylated in the pitch to produce phenolic antioxidants in the pitch itself.

2. A method for producing an antioxidant from a phenolic pitch including phenol (III), 2- and 4-cumylphenol (V), (VI), high-molecular-weight phenolic derivatives (XIII) and aromatic compounds of a non-phenolic nature including acetophenone (VII), dimethylphenylcarbinol (VIII), 2,4-diphenyl-4-methyl-1-pentene (IX), 2,4-diphenyl-4-methyl-2-pentene (X), α-methyl styrene (XI), characterised in that the phenol (III), the 2-cumylphenol (V), the 4-cumylphenol (VI) and the high-molecular-weight phenolic derivatives (XIII) are alkylated in the pitch by means of the aromatic compounds of a non-phenolic nature (VIII), (IX), (X), (XI) to produce phenolic antioxidants in the pitch itself.

3. A method according to Claim 1 or Claim 2, characterised in that the alkylation is carried out after the addition to the phenolic pitch of at least one compound selected from the group comprising: olefins, chloroparaffins, alcohols, ethers, possibly substituted with one or more aromatic groups and having from 4 to 18 carbon atoms.

4. A method according to Claim 3, characterised in that the alkylating compound is selected from the group comprising: isobutene, isopentene, tert-butyl alcohol, tert-butyl chloride, methyl tert-butyl ether, styrene, methylphenylcarbinol.

5. A method according to Claim 4, characterised in that the methylphenylcarbinol is produced in the

phenolic pitch by the selective reduction of the acetophenone (VII).

6. A method according to Claim 5, characterised in that the selective reduction is carried out in the pitch before the alkylation.

7. A method according to Claim 5, characterised in that the acetophenone (VII) reduction stage is carried out beforehand by means of the addition of $NaBH_4$ to the pitch in a proportion of 5-7% by weight, at a temperature of from 25-30° C, over a period of approximately 2 hours.

8. A method according to Claim 5, characterised in that the reduction stage is effected by means of an hydrogenation catalyst, preferably selected from the group comprising Raney nickel and platinum black.

9. A method according to Claim 2, characterised in that the acetophenone (VII) is separated from the phenolic pitch before the alkylation.

10. A method according to Claim 2, characterised in that the acetophenone (VII) is separated from the phenolic antioxidant after the alkylation.

11. A method according to Claim 9 or Claim 10, characterised in that the separation is carried out by distillation under vacuum.

12. A method according to Claim 1 and Claim 2, characterised in that the alkylation is a Friedel-Crafts alkylation.

13. A method according to Claim 12, characterised in that the Friedel-Crafts alkylation is carried out after the addition of aluminium chloride as a catalyst.

14. A method according to Claim 13, characterised in that the aluminium chloride is added to the phenolic pitch in a quantity of between approximately 2 and 10%.

15. A method according to Claims 12 to 14, characterised in that the Friedel-Crafts alkylation is carried out at a temperature between 25 and 100° C and for a period which ranges from approximately 24 hours to approximately 3 hours respectively.

16. A method according to one of the preceding claims, characterised in that the phenolic pitch is a pitch residue resulting from the production of phenol from cumene.

17. An antioxidant produced from a phenolic pitch residue resulting from the production of phenol from cumene, characterised in that it includes phenolic antioxidants having the following structural formulae:

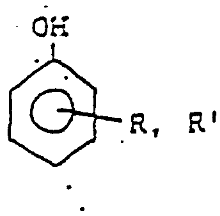

(XIV)

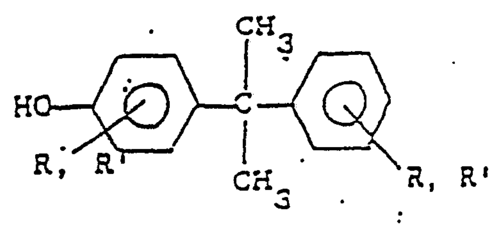

(XV)

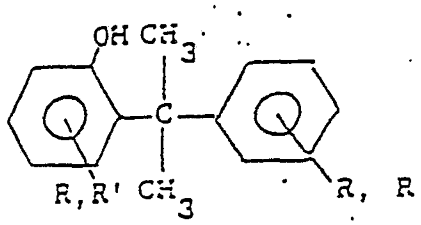

(XVI)

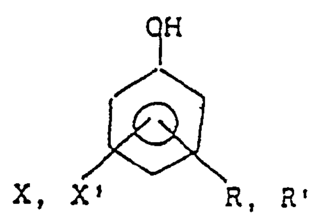

(XVII)

where X, X′ comprise, independently, hydrogen and at least one substituent selected from the group comprising: saturated or unsaturated linear or branched alkyl groups with from 2 to 6 alkyl carbon atoms, aryl groups having from 6 to 18 aromatic carbon atoms, possibly substituted with hydroxylic groups and/or linear or branched alkyl groups with from 2 to 6 carbon atoms; R, R′ comprise, independently, hydrogen and at least one substituent selected from the group comprising: isopropyl, sec-butyl, tert-butyl, tert-amyl, sec-ethylbenzyl and the groups having the following structural formulae:

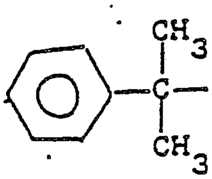

(XVIII)

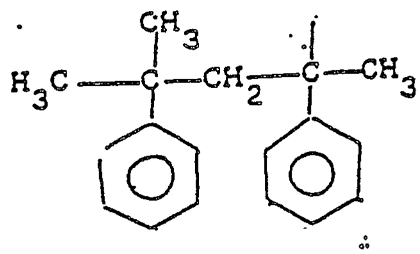

(XIX)

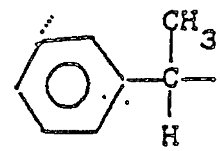

(XXI)